# EUROPEAN PATENT SPECIFICATION

(11) **EP 1 133 517 B1**
(45) Date of publication and mention of the grant of the patent: **22.07.2009**
(21) Application number: 99960422.6
(22) Date of filing: 17.11.1999
(51) Int. Cl.: C07K 1/00, C07K 14/00, C07K 14/47, A61K 38/17

(54) **INTRANUCLEAR TARGETED DELIVERY OF COMPOUNDS BY 70 kD HEAT SHOCK PROTEIN**
INTRANUKLEÄRER, GERICHTETER TRANSPORT VON VERBINDUNGEN DURCH DAS 70KD-HITZESCHOCKPROTEIN
TRANSPORT INTRANUCLEAIRE CIBLE DE COMPOSES PAR LA PROTEINE DE CHOC THERMIQUE 70 kD

(30) Priority: 24.11.1998 US 109872 P
(43) Date of publication of application: 19.09.2001
(73) Proprietor: Bristol-Myers Squibb Company, Princeton NJ 08543-4000 (US)
(72) Inventor: FUJIHARA, Sheri, M., San Leandro, CA 94579 (US); NADLER, Steven, G., Princeton, NJ 08540 (US)
(74) Representative: Vossius & Partner
(86) International application number: PCT/US1999/027244
(87) International publication number: WO 2000/031113

(56) References cited:
- WO-A-94/29459
- BLACHERE NATHALIE E ET AL: "Heat shock protein-peptide complexes, reconstituted in vitro, elicit peptide-specific cytotoxic T lymphocyte response and tumor immunity." JOURNAL OF EXPERIMENTAL MEDICINE, vol. 186, no. 8, 1997, pages 1315-1322, XP002091660 ISSN: 0022-1007
- CIUPITU ANNE-MARIE T ET AL: "Immunization with a lymphocytic choriomeningitis virus peptide mixed with heat shock protein 70 results in protective antiviral immunity and specific cytotoxic T lymphocytes." JOURNAL OF EXPERIMENTAL MEDICINE, vol. 187, no. 5, 2 March 1998 (1998-03-02), pages 685-691, XP002143653 ISSN: 0022-1007
- FUJIHARA SHERI M ET AL: "Intranuclear targeted delivery of functional NF-kappaB by 70 kDa heat shock protein." EMBO (EUROPEAN MOLECULAR BIOLOGY ORGANIZATION) JOURNAL, vol. 18, no. 2, 15 January 1999 (1999-01-15), pages 411-419, XP002219272 ISSN: 0261-4189
- TAKAKURA ET AL: 'Macromolecular carrier systems for targeted drug delivery: pharmacokinetic consideration on biodistribution' PHARMACEUTICAL RESEARCH vol. 13, no. 6, 1996, pages 820 - 831, XP002923301
- MARGULIS ET AL: 'Liposomal delivery of purified heat shock protein hsp70 into rat pancreatic islets as protection against interleukin 1beta-induced impaired beta-cell function' DIABETES vol. 40, November 1991, pages 1418 - 1422, XP002923302
- DANG ET AL: 'Nuclear and nucleolar targeting sequences of c-erb-A, c-myh, N-myc, p53, HSP70 and HIV tat proteins' THE JOURNAL OF BIOLOGICAL CHEMISTRY vol. 264, no. 30, 25 October 1989, pages 18019 - 18023, XP002923303
- PERKINS ET AL: 'Distinct combination of NF-kB subunits determine the specificity of transcriptional activation' PROC. NATL. ACAD. SCI. USA, vol. 89, March 1992, pages 1529 - 1533, XP002923304
- LECLAIR ET AL: 'The p50 subunit of NF-kB associates with the NF-IL6 transcription factor' PROC. NATL. ACAD. SCI. USA, vol. 89, September 1992, pages 8145 - 8149, XP002923305
- JOHNSON ET AL: 'Exogenous HSP70 becomes cell associated, but not internalized by stressed arterial smooth muscle cells' IN VITRO CELLULAR AND DEVELOPMENTAL BIOLOGY vol. 29A, October 1993, pages 807 - 812, XP002923306
- FIX J.A.: 'Oral controlled release technology for peptides: status and future prospects' PHARMACEUTICAL RESEARCH vol. 13, no. 12, 1996, pages 1760 - 1764, XP002923307

## Description

### Field of the Invention

The present invention relates to the intracellular delivery, preferably the intranuclear delivery, of compounds using the heat shock protein Hsp70.

### Background of the Invention

Heat shock proteins ("Hsps") are a family of molecular chaperone proteins which have long been known to play essential roles in a multitude of intra- and intercellular processes, including protein synthesis and folding, vesicular trafficking, and antigen processing and presentation. Hsps are among the most highly conserved proteins known, and carry out many of their regulatory activities via protein-protein interactions. Hsp70 is one member of the heat shock protein family. (Milner, C.M. and Campbell, R.D. Immunogenetics 32:242-251 (1990); Genbank Accession No. M59828). One of the most well characterized functions of Hsp70 is to assist in the translocation of proteins across intracellular membranes into different compartments of the cell.

Intracellular transport activity has been reported for viral proteins such as the HSV-1 structural protein VP22 (Elliott, et. al., (1997) Cell 88:223-233) and the HIV Tat protein (Vives, et. al., (1997) J. Biol. Chem. 272:16010-16017), as well as peptide sequences derived from Antennapedia homeodomain, fibroblast growth factor (Hawiger, (1997) Curr. Opin. Immun. 9:189-194), and most recently the neuropeptide galanin (Pooga, et. al., (1998) FASEB J. 12:67-77). Delivery of protein substrates has been demonstrated by some of these transport peptides as well (Phelan, et. al., (1998) Nature Biotechnology 16:440-443; Fawell, et. al., (1994) Proc. Natl. Acad. Sci. 91:664-668; Rojas, et. al., (1998) Nature Biotechnology 16:370-375).

Hsps also serve a number of key functions in the immune response, and over the past few years there has been increasing interest in characterizing the nature of Hsps in generating protective immunity. A series of recent studies (Roman, et. al., (1996) Immunology 88:487-492; Suzue, et. al., (1996) J. Immunol. 156:873-879) demonstrated that Hsp70 could act as a carrier protein to enable a bound peptide or protein substrate to enter the endosomal compartment and subsequently access the MHC class II processing pathway for exogenous antigens. Such treatment with Hsp70-peptide complexes or Hsp70 fusion proteins could elicit cargo-specific proliferative T cell responses. However, other experiments in which cancer cell-derived Hsp70 used to immunize mice resulted in specific antitumor CTL responses (Udono, et al., (1993) J. Exp. Med. 178:1391-1396) suggested that the Hsps were using the endogenous MHC class I processing pathway. These data implied that Hsp70 was able to cross the plasma membrane and enter the cytoplasmic compartment of intact cells. Earlier studies (Hightower, et al. (1989) J. Cell Plysiol. 138:257-266) reporting the release of Hsps from axonal cells by a non-heat shock dependent mechanism, support the observation that some Hsp family members can cross the plasma membranes of certain cells.

Although the above studies imply a plasma membrane translocation capacity for Hsp70, such an activity has not been directly demonstrated. It has not been shown whether or not Hsp70 could be utilized to deliver proteins across the plasma and nuclear membranes. There exists a need to deliver compounds, such as proteins or DNA, into the cell nucleus to modulate cellular activity.

Applicants have shown that the human 70 kD heat shock protein can translocate across cell membranes to rapidly gain cytoplasmic and nuclear entry. Furthermore, chimeric proteins composed of Hsp70 peptides fused to amino acids 37-409 of the p50 subunit of NF-κB (Meyer, R., et. al., PNAS 88:966-970 (1991), Genbank Accession No. M58603) also exhibit this translocation property. Though cellular import activity has been reported for various diverse peptides, intranuclear transport generally requires the presence of specific nuclear localization sequences ("NLS"). While heat shock is known to induce increased synthesis and nuclear translocation of endogenous heat shock protein following heat shock factor activation, Applicants show that nuclear localization of exogenous heat shock protein can result without prior heat shock. Applicants have also shown that the transport and nuclear localization properties of Hsp70 are retained within a 90 amino acid C-terminal fragment; successful intranuclear transport has been demonstrated utilizing a C-terminal fragment as well as larger fragments encompassing more of the peptide binding domain. Herein is the first evidence establishing the ability of exogenous Hsp70 fusion proteins to cross the cell membrane, gain nuclear entry and exert a biological function.

Therefore, an object of the present invention is to provide a carrier for delivery of molecules with biological function into both cellular and nuclear compartments. A preferred embodiment of the present invention utilizes Hsp70, or a fragment of Hsp70 as described herein, as a vehicle for directed, noninvasive delivery of molecules, such as proteins or DNA, that may modulate gene expression.

### Summary of the Invention

The 70kD heat shock protein ("Hsp70") is a highly conserved, ubiquitous protein involved in chaperoning proteins to various cellular organelles. Applicants herein show that when added exogenously to cells, Hsp70 is readily imported into both cytoplasmic and nuclear compartments. Applicants have demonstrated that Hsp70 can be used to chaperone compounds into a cell or cells. Hsp70 was used to deliver NF-κB, a key transcriptional regulator of inflammatory responses, into the nuclear compartment. Applicants herein show that a fusion protein composed of a C-terminal Hsp70 peptide and amino acids 37-409 of the p50 subunit of NF-κB was directed into the nucleus of cells, could bind DNA specifically, and activated kappa Ig expression and TNFα production. Applicants' invention encompasses the use of Hsp70 as a vehicle for intracytoplasmic and intranuclear delivery of proteins or DNA to modulate gene expression and thereby control immune responses.

### Description of the Figures

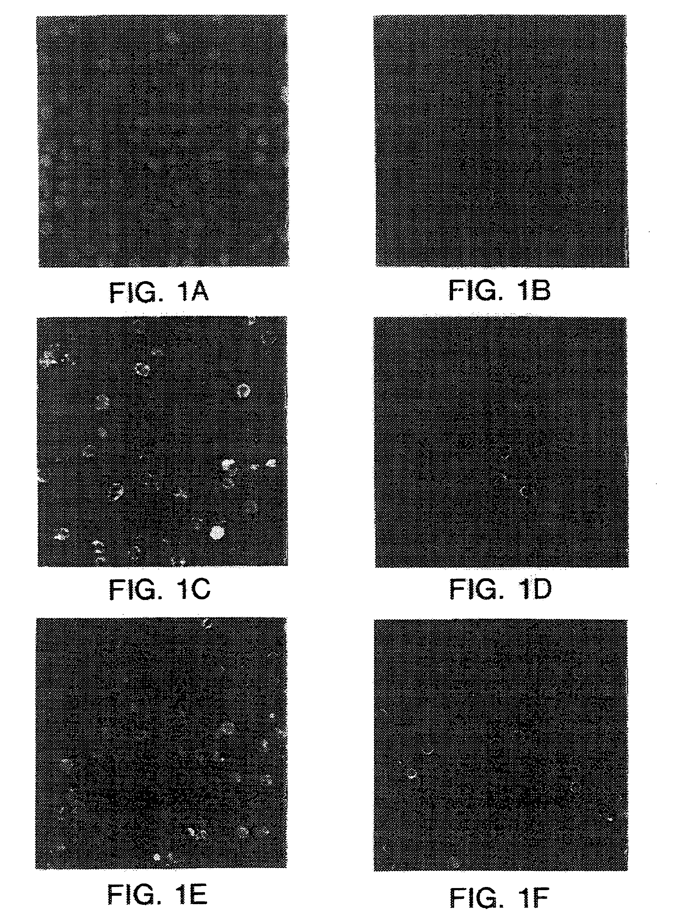

Figure 1 shows various cell types that exhibit differential Hsp70 uptake activity. Various cells were treated with 10µg/gml Hsp70-FITC added to the culture media. Human peripheral blood cells were stained with anti-CD 14-PE as a marker for monocytic cells, anti-CD19-PE for B cells, or anti-CD3-PE for T cells. After one hour of incubation at 37°C, cells were washed in PBS, fixed in 2% paraformaldehyde, washed again and re-suspended in PBS for visualization by confocal laser scanning microscopy. Equimolar amounts of BSA-FITC were used in parallel experiments as a control. Figure 1A shows 70Z/3 cells + Hsp70-FITC; Figure 1B shows 70Z/3 cells + BSA-FITC; Figure 1C shows PBL stained with anti-CD14-PE + Hsp70-FITC; Figure 1D shows PBL stained with anti-CD14-PE + BSA-FITC; Figure 1E shows PBL stained with anti-CD 19-PE + Hsp70-FITC; and Figure IF shows peripheral blood T cells stained with anti-CD3-PE + Hsp70.

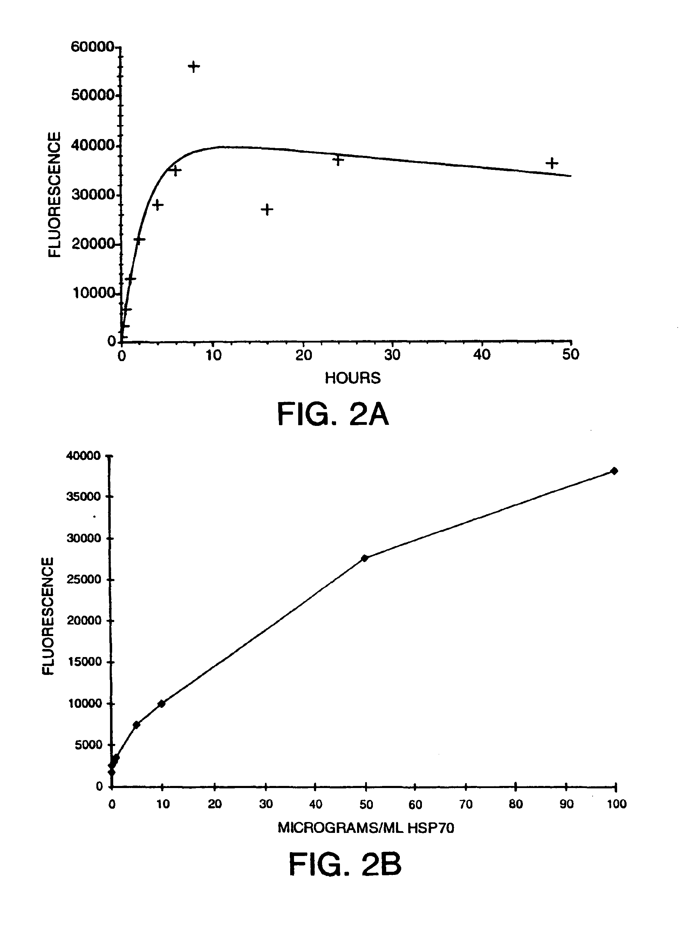

Figure 2 shows the kinetics of Hsp70 cellular uptake. Figure 2A shows the kinetics of uptake of Hsp70-FITC by 70Z/3 cells. The cells were incubated at 37°C for various times with 1 uM Hsp70-FITC in complete RPMI. Cells were washed once in PBS to separate free Hsp70-FITC, then re-suspended in PBS and analyzed by fluorimeter. Points were experimental and the curve was fitted by a modified regression program (XLlfit). Figure 2B shows the dose effect of Hsp70 on uptake by 70Z/3 cells. The cells were incubated at 37°C for one hour with various concentrations of Hsp70-FITC, then washed and analyzed as in (A).

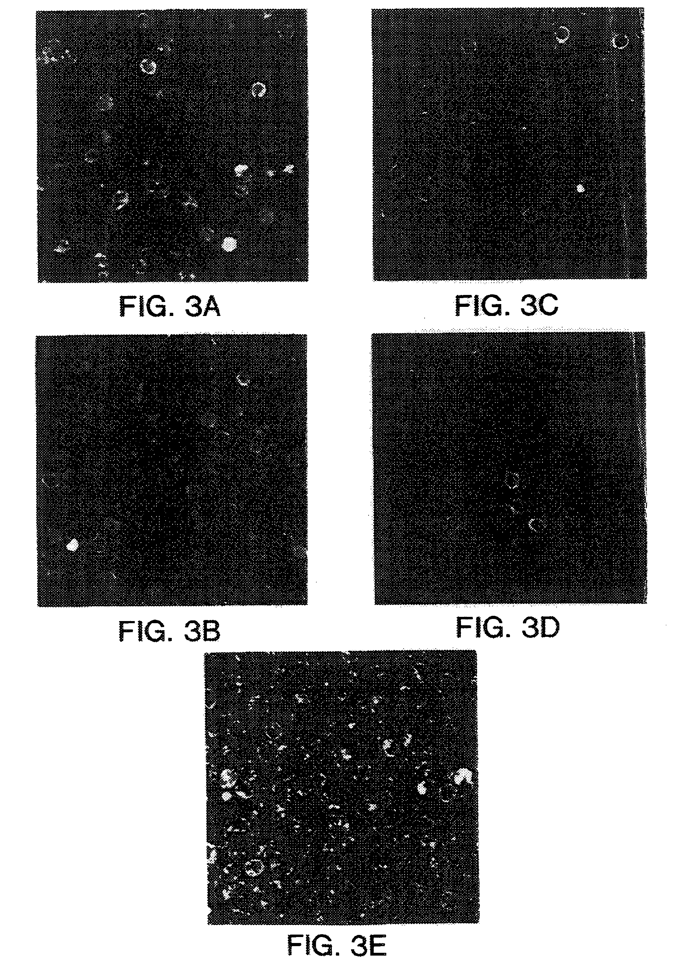

Figure 3 shows that intracellular uptake of Hsp70-FITC was not affected by azide but was inhibited at 4°C. 70Z/3 cells were either untreated (Figure 3A) or pretreated for 30 minutes with 0.05% sodium azide (Figure 3B) before incubation with Hsp70-FITC at 37°C, or were preincubated at 4°C for 30 minutes prior to addition of Hsp70-FITC and an additional one hour of incubation at 4°C (Figure 3C). BSA-FITC was added to cells for 1 hour at 37°C as a control (Figure 3D). Internalized Hsp70-FITC did not co-localize with transferrin. Cells were treated with both Hsp70-FITC and Texas Red-conjugated transferrin, for one hour at 37°C (Figure 3E).

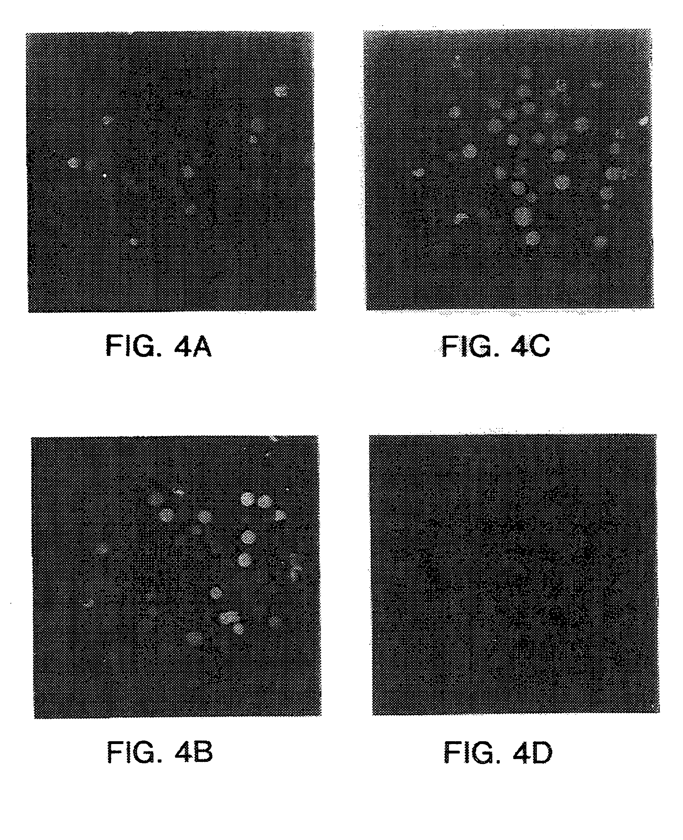

Figure 4 shows transport of fusion proteins into the cytoplasm and nucleus. FITC-labeled fusion proteins consisting of either the C terminal 244 (Hsp70/28-p50) or 92 (Hsp70/10-p50) amino acids of Hsp70, fused to amino acids 37-409 of the p50 subunit of NF-kB, were transported into 70Z/3 cells. 70Z/3 cells were treated with full-length Hsp70-FITC (Figure 4A), Hsp70/28-p50-FITC (Figure 4B), Hsp70/10-p50-FITC (Figure 4C), or BSA-FITC as a control (Figure 4D) for 1 hour at 37°C as described. Intracellular localization of fusion proteins was visualized by confocal laser scanning microscopy.

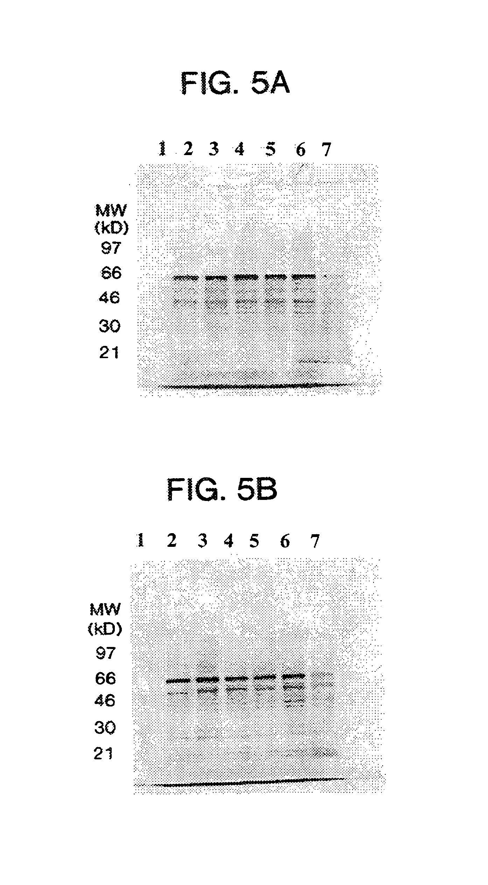

Figure 5 demonstrates that internalized intracellular Hsp70 or Hsp70-p50 remained stable for up to 24 hours. Cells were treated with either full-length Hsp70-FITC (Figure 5A) or Hsp70/28-p50-FITC (Figure 5B) for one hour prior to washing and additional incubation at 37°C for increasing times. Cells were harvested at the indicated timepoints, lysed in Laemmli sample buffer, and whole cell lysate proteins were separated by SDS-PAGE. Gels were subjected to fluorimager analysis. Lanes 1: cells untreated; lane 2: no chase; lane 3: 1 hour of chase; lane 4: 2 hours of chase; lane 5: 6 hours of chase; lane 6: 24 hours of chase; lane 7: 4 days of chase.

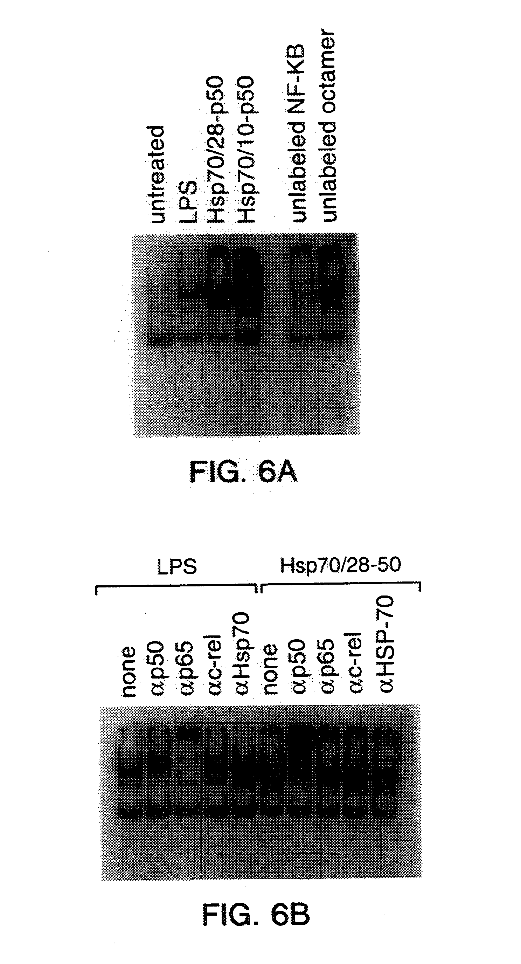

Figure 6 shows that internalized Hsp70-p50 fusion proteins exhibited DNA-binding activity. 70Z/3 cells were treated as indicated for one hour prior to lysis and generation of nuclear extracts. EMSA was performed and specific DNA binding complexes were identified by supershift assay with the indicated antibodies. Figure 6A, lane 1: unstimulated cells control; lane 2: LPS-treated; lane 3: Hsp70-p50-treated; lane 4: Hsp70-p50-treated extracts competed with unlabelled NF-κB oligo; lane 5: same as lane 4 but competed with unlabelled octamer oligo. Figure 6B, lane 1: LPS-treated; lane 2: LPS-treated and supershifted with anti-p50; lane 3: anti-p65; lane 4: anti-c-rel; lane 5: anti-Hsp70; Lanes 6-10, same as lanes 1-5 but using Hsp70-p50 treated extracts.

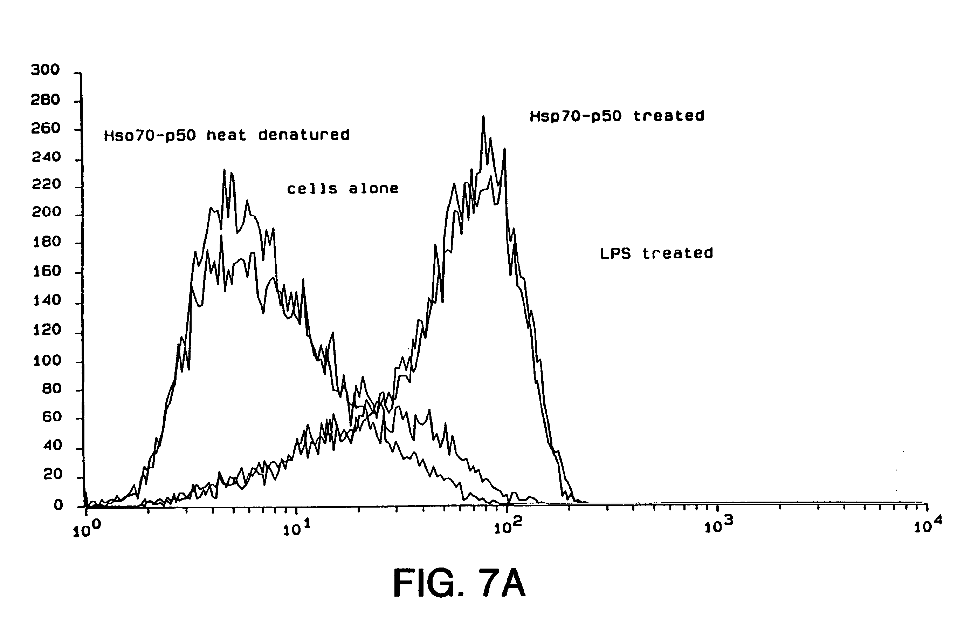

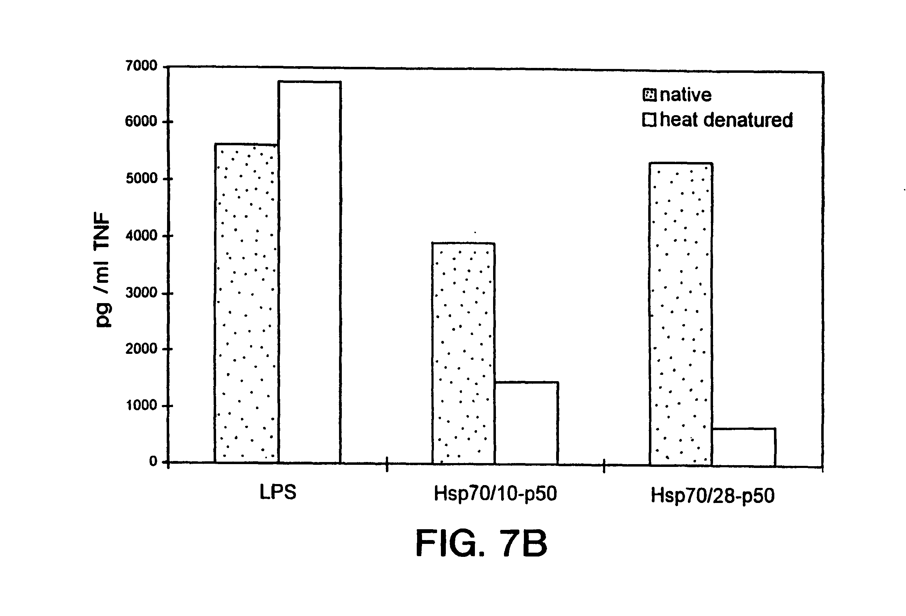

Figure 7 shows that Hsp70-p50-treated cells became activated to express surface kappa Ig and produce TNFα. (Figure 7A) 70Z/3 cells were treated with 10 ng/ml LPS or 30 µg/ml Hsp70/ 10-p50 overnight prior to washing and staining with anti-kappa-FITC and FACS analysis. (Figure 7B) Human peripheral blood lymphocytes were treated with 5 ng/ml LPS or 40 µg/ml Hsp70/10-p50 or Hsp70/28-p50 for 6 hours, and supernatants were harvested and analyzed for TNFα levels by ELISA.

### Detailed Description of the Invention

The present invention discloses an in vitro method for delivering a compound selected from the group consisting of a protein, a peptide, a polynucleotide or a therapeutic agent into the nucleus of a cell or cells, said method comprising joining said compound with Hsp70, or a fragment of Hsp70 comprising an amino acid sequence as shown in SEQ ID NO: 2 or SEQ ID NO: 3, to form an Hsp70 complex, and exogenously providing said Hsp70 complex to said cell or to the environment surrounding said cell so that Hsp70 complex is imported into the nucleus of said cell or cells.

Disclosed is the use of a Hsp70 complex for the preparation of a pharmaceutical composition for treating transplant rejection, an autoimmune disease, an inflammatory disease, cancer, or a vascular disease in a patient, wherein a compound is covalently joined with Hsp70, or a fragment of Hsp70 comprising an amino acid sequence as shown in SEQ ID NO: 2 or SEQ ID NO: 3, to form the Hsp70 complex and wherein said complex is to be imported into the nucleus of a cell or cells.

The present invention further provides a pharmaceutical composition comprising an Hsp70 complex, said Hsp70 complex comprising a first portion and second portion, said first portion comprising Hsp70 protein, or a fragment of Hsp70 comprising an amino acid sequence as shown in SEQ ID NO: 2 or SEQ ID NO: 3; and said second portion comprising a nuclear protein, nuclear peptide, or polynucleotide.

The present invention demonstrates that the heat shock protein Hsp70 is internalized by cells into both the cytoplasm and nucleus in a cell type specific manner. Although the mechanism of uptake is unknown, the data suggest that the binding and internalization of Hsp70 is energy dependent and involves a high capacity receptor.

The observation that cell surface-associated and secreted forms of Hsp70 exist (Multhoff, et al., (1996) Cell Stress & Chaperones 1:167-176) suggests that this protein may function in cell-cell communication, perhaps as a means of transferring cellular protection from environmental stressors by regulating transcription. In fact, several recent reports have described a physical interaction of Hsp70 with the transactivation domains of several transcription factors within the nucleus. For example, Hsp70 can bind directly to the transactivation domains of both HSF (heat shock factor) (Shi, et al., (1998) Genes Dev. 12:654-666), resulting in inhibition of gene transcription, and Wilms tumor suppressor (Maheswaran, et al., (1998) Genes Dev. 12:1108-1120), resulting in suppression of cellular proliferation. These data and others implicate a role for Hsp70 in the regulation of transcription factors and possibly other nuclear proteins. These data complement others which have shown that Hsp70 as a cytoplasmic chaperone can interact with transcription factors such as NF-κB itself, as well as a myriad cofactors such as Hip, Hop, Hsp40, Hsp90, BAG-1 and others (Demand, et al., (1998) Mol. Cell. Biol. 18:2023-2028). Indeed, release and intercellular transfer of exported Hsp70 has been reported in glial and axonal cells (Hightower, et al., (1989) J. Cell Physiol. 138:257-266); and accumulation of Hsp70 in a variety of human cell lines either by heat shock or by liposomal transfer has been shown to increase cell survival and protect from apoptotic cell death (Lasunskaia, et al., (1997) Apoptosis 2:156-163). Release of heat shock proteins from cells under harsh or damaging conditions may be a homeostatic mechanism for transfer of a protective stress response to neighboring cells that are unable to mount such a response. In addition, recent reports describing the ability of peptide-bound Hsp70 molecules to induce antitumor or antiviral immunity as well as development of memory CTLs support the notion that these proteins might function to convey a protective immune response by providing an antigen presentation function (Blachere, et al., (1997) J. Exp. Med. 186:1315-1322; Ciupitu, et al., (1998) J. Exp. Med. 187:685-691). Applicants submit that endogenous Hsp70 (and associated peptides or proteins) is/are released into the environment by infected or apoptosing cells. These Hsp70 protein complexes would subsequently become available to neighboring cells which may be compromised in their immune capacity, and act as a stimulus to boost or strengthen the immune response.

Applicants have demonstrated the efficient cellular and nuclear uptake as well as long-term intracellular stability of exogenously supplied Hsp70 fusion proteins by a variety of cell types. This rapid and stable transport activity has important implications for the utility of Hsp70-derived peptides as a vehicle for delivering therapeutic agents to the cytoplasm and nucleus where they remain localized for long periods of time. As manipulation of the nuclear import process in particular becomes an increasingly more interesting target for regulated control of gene expression (Fujihara, et al., (1998) Biochem. Pharm. 56:157-161), it is believed that future emphasis will be placed on developing more potent means of intracellular targeted delivery. The use of Hsp70 as a delivery system has a number of advantages over other previously described protein candidates, including the fact that the protein is of human origin and therefore does not contain foreign (i.e., viral or insect) and potentially immunogenic material. Use of soluble fragments of Hsp70 will potentially reduce immunogenicity further. As Hsp70 is a highly expressed abundant protein, it would likely be well-tolerated in humans, and in fact already plays an immune response role. In addition, the cell type-specificity we observed would allow the targeting of compounds to specific cells of the immune system for more effective regulatory control. And finally, the preferential and long-lived nuclear directed delivery of protein substrates may provide protection from cytoplasmic proteolysis. Our data support the potential use of Hsp70 sequences as a novel tool to deliver molecules that modulate gene expression and subsequently provide immunosuppressive or immunostimulatory control.

Applicants disclose in an embodiment of the present invention a fusion protein comprising a fragment of Hsp70 joined to amino acids 37-409 of the NF-κB p50 subunit (the fusion proteins are referred to herein as Hsp70-p50, Hsp70/28-p50, or Hsp70/10-p50). Although NF-κB p50 homodimers are commonly thought to act as NF-κB transcriptional repressors in most cell types due to the absence of a C-terminal activation domain, the downstream biological events Applicants observed did not reflect a dominant negative effect of the fusion protein on transcriptional activation. There are a number of possible explanations for the Hsp70-p50-induced transactivation observed. First, the heat shock protein sequence, specifically the EEVD domain, may itself contain transactivation activity. Hsp70 is known to bind heat shock factor in the nucleus and interfere with its transactivation activity via the EEVD domain. Since the Hsp70 sequence in the Examples below was cloned C-terminal to amino acids 37-409 of the NF-kB p50 sequence (SEQ ID NO:1), the EEVD domain is potentially available to provide transactivation, or even to interact with other cellular or nuclear cofactors. Closer analysis of the nuclear complex may yield clues as to other possible components with transcriptional activities. Second, p50 homodimers may simply exhibit transactivation activity in particular circumstances. Fujita, et al. ((1992) Genes Dev. 6:775-787) tested the various homo-and heterodimers of NF-κB subunits for transcriptional activation in vitro and determined that addition of p50 alone to some transcription mixtures resulted in significant transcriptional stimulation. They attribute this activation to differences in the fine structure of the nucleotide sequence within the κB motifs. Interestingly, this group observed a four-fold stimulation of transcription by p50 homodimers over control using the Igκ sequence motif. These data would correspond to the activation we observed in studies analyzing surface kappa Ig expression. Third, the Hsp70-p50 subunits may be recruiting other transactivating factors into the DNA binding complex that we have not yet detected.

Therefore the present invention encompasses the use of Hsp70, or a fragment of Hsp70 comprising an amino acid sequence as shown in SEQ ID NO:2 or SEQ ID NO:3; to modulate cellular activity, preferably modulate nuclear activity in a cell or cells, for example the activity of transcription factors. The term "nuclear activity" encompasses the transcription of nucleic acid molecules in the cell. The term "modulate" encompasses enhancement, diminishment, activation or inactivation of cellular activity. The Hsp70 protein or a fragment thereof as defined above may be used alone to modulate cellular activity by transfer into the cytoplasm and/or nucleus of a cell, to treat Hsp70-associated disorders. "Hsp70-associated disorders" refers to any disorder or disease state in which Hsp70 plays a regulatory role in the metabolic pathway of that disorder or disease. As used herein the term "treating" refers to the alleviation of symptoms of a particular disorder in a patient, the improvement of an ascertainable measurement associated with a particular disorder, or the prevention of a particular immune response (such as transplant rejection). The term "patient" refers to a mammal, preferably a human.

Additionally, the present invention encompasses the use of Hsp70, or a fragment of Hsp 70 as defined above, as a chaperone to carry one or more compounds into a cell. Hsp70 or a fragment thereof is joined to a compound to form a complex (herein referred to as an "Hsp70 complex" which includes the Hsp70, or fragment as defined above thereof, and any compound associated with or joined to the Hsp70 protein or fragment as defined above thereof). The Hsp70 complex is then provided to a cell or cells, or to the environment surrounding a cell or cells, so that the Hsp70 complex is transported into the cytoplasm and/or nucleus of the cell or cells. Compounds that may be joined to the Hsp70 protein or a fragment as defined above thereof include, but are not limited to, proteins, peptides, nucleic acids, and small molecules. "Nucleic acids" or "polynucleotides" includes individual nucleotides as well as DNA and RNA sequences or fragments thereof.

Disease states which may be treated by Hsp70, fragments as defined above thereof, and/or Hsp70 complexes of the present invention include transplant rejection and autoimmune diseases, such as rheumatoid arthritis, multiple sclerosis, juvenile diabetes, asthma, and inflammatory bowel disease, as well as inflammatory diseases, cancer, viral replication diseases and vascular diseases.

For example, the Hsp70 complexes and pharmaceutical compositions of the present invention are useful in the treatment of transplant rejection (e.g., kidney, liver, heart, lung, pancreas (e.g., islet cells), bone marrow, cornea, small bowel and skin allografts, and heart valve xenografts) and autoimmune diseases, such as rheumatoid arthritis, multiple sclerosis, juvenile diabetes, asthma, inflammatory bowel disease (Crohn's disease, ulcerative colitus), lupus, diabetes, myasthenia gravis, psoriasis, dermatitis, eczema, seborrhoea, pulmonary inflammation, eye uveitis, hepatitis, Grave's disease, Hashimoto's thyroiditis, Behcet's or Sjorgen's syndrome (dry eyes/mouth), pernicious or immunohaemolytic anaemia, idiopathic adrenal insufficiency, polyglandular autoimmune syndrome, glomerulonephritis, scleroderma, lichen planus, viteligo (depigmentation of the skin), autoimmune thyroiditis, and alveolitis, inflammatory diseases such as osteoarthritis, acute pancreatitis, chronic pancreatitis, asthma and adult respiratory distress syndrome, as well as in the treatment of cancer and tumors, such as solid tumors, lymphomas and leukemia, vascular diseases such as restenosis, stenosis and artherosclerosis.

Also within the scope of the present invention are pharmaceutical compositions comprising at least one Hsp70 complex comprising a compound that is to be delivered to the cytoplasm and/or nucleus of a cell or cells. The Hsp70 complex may be administered alone or with at least one additional active compound, and any pharmaceutically acceptable carrier, adjuvant or vehicle. "Additional active compounds" encompasses an agent or agents selected from the group consisting of an immunosuppressant, an anti-cancer agent, an anti-viral agent, an anti-inflammatory agent, an anti-fungal agent, an antibiotic, or an anti-vascular hyperproliferation compound.

The term "pharmaceutically acceptable carrier, adjuvant or vehicle" refers to a carrier, adjuvant or vehicle that may be administered to a subject, together with an Hsp70 complex of the present invention, and which does not destroy the pharmacological activity thereof. Pharmaceutically acceptable carriers, adjuvants and vehicles that may be used in the pharmaceutical compositions of the present invention include the following: ion exchangers, alumina, aluminum stearate, lecithin, self-emulsifying drug delivery systems ("SEDDS") such as dαtocopherol polyethyleneglycol 1000 succinate, surfactants used in pharmaceutical dosage forms such as Tweens® or other similar polymeric delivery matrices, serum proteins such as human serum albumin, buffer substances such as phosphates, glycine, sorbic acid, potassium sorbate, partial glyceride mixtures of saturated vegetable fatty acids, water, salts or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Cyclodextrins such as α-, p- and γ-cyclodextrin, or chemically modified derivatives such as hydroxyalkylcyclodextrins, including 2- and 3-hydroxypropyl-β-cyclodextrins, or other solubilized derivatives may also be used to enhance delivery of the compounds of the present invention.

The compositions of the present invention may contain other therapeutic agents as described below, and may be formulated, for example, by employing conventional solid or liquid vehicles or diluents, as well as pharmaceutical additives of a type appropriate to the mode of desired administration (for example, excipients, binders, preservatives, stabilizers, flavors, etc.) according to techniques such as those well known in the art of pharmaceutical formulation.

Pharmaceutical compositions comprising at least one Hsp70 complex of the present invention may be administered by any suitable means, for example, orally, such as in the form of tablets, capsules, granules or powders; sublingually; buccally; parenterally, such as by subcutaneous, intravenous, intramuscular, or intrasternal injection or infusion techniques (e.g., as sterile injectable aqueous or non-aqueous solutions or suspensions); nasally such as by inhalation spray; topically, such as in the form of a cream or ointment; or rectally such as in the form of suppositories; in dosage unit formulations containing non-toxic, pharmaceutically acceptable vehicles or diluents. The pharmaceutical compositions of the present invention may, for example, be administered in a form suitable for immediate release or extended release. Immediate release or extended release may be achieved by the use of suitable pharmaceutical compositions comprising the present compounds, or, particularly in the case of extended release, by the use of devices such as subcutaneous implants or osmotic pumps. The present Hsp70 complexes may also be administered liposomally.

Exemplary compositions for oral administration include suspensions which may contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which may contain, for example, microcrystalline cellulose, dicalcium phosphate, starch, magnesium stearate and/or lactose and/or other excipients, binders, extenders, disintegrants, diluents and lubricants such as those known in the art. The present compounds may also be delivered through the oral cavity by sublingual and/or buccal administration. Molded tablets, compressed tablets or freeze-dried tablets are exemplary forms which may be used. Exemplary compositions include those formulating the present Hsc70 complexes with fast dissolving diluents such as mannitol, lactose, sucrose and/or cyclodextrins. Also included in such formulations may be high molecular weight excipients such as celluloses (avicel®) or polyethylene glycols (PEG). Such formulations may also include an excipient to aid mucosal adhesion such as hydroxy propyl cellulose (HPC), hydroxy propyl methyl cellulose (HPMC), sodium carboxy methyl cellulose (SCMC), maleic anhydride copolymer (e.g., Gantrez®), and agents to control release such as polyacrylic copolymer (e.g., Carbopol® 934). Lubricants, glidants, flavors, coloring agents and stabilizers may also be added for ease of fabrication and use.

Exemplary compositions for nasal aerosol or inhalation administration include solutions in saline which may contain, for example, benzyl alcohol or other suitable preservatives, absorption promoters to enhance bioavailability, and/or other solubilizing or dispersing agents such as those known in the art.

Exemplary compositions for parenteral administration include injectable solutions or suspensions which may contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid. The term "parenteral" as used herein includes subcutaneous, intracutaneous, intravenous, intramuscular, intraarticular, intraarterial, intrasynovial, intrasternal, intrathecal, intralesional and intracranial injection or infusion techniques.

Exemplary compositions for rectal administration include suppositories which may contain, for example, a suitable non-irritating excipient, such as cocoa butter, synthetic glyceride esters or polyethylene glycols, which are solid at ordinary temperatures, but liquify and/or dissolve in the rectal cavity to release the drug.

Exemplary compositions for topical administration include a topical carrier such as Plastibase™ (mineral oil gelled with polyethylene).

A "therapeutically effective" amount of an Hsp70 complex of the present invention may be determined by one of ordinary skill in the art, and includes exemplary dosage amounts for an adult human of from about 0.1 to 100 mg/kg of body weight of active compound per day, which may be administered in a single dose or in the form of individual divided doses, such as from 1 to 3 times per day. It will be understood that the specific dose level and frequency of dosage for any particular subject may be varied and will depend upon a variety of factors including the activity of the specific compound employed, the metabolic stability and length of action of that compound, the species, age, body weight, general health, sex and diet of the subject, the mode and time of administration, rate of excretion, drug combination, and severity of the particular condition. Subjects to be treated may include animals, or mammalian species such as humans.

By "therapeutically effective" is meant an amount necessary to achieve a desired result, for example, alleviation of symptoms of a particular disorder in a patient, the improvement of an ascertainable measurement associated with a particular disorder, or the prevention of a particular immune response. It will be understood that the specific dose level and frequency of dosage for any particular subject may be varied and will depend upon a variety of factors including the activity of the specific compound employed, the metabolic stability and length of action of that compound, the species, age, body weight, general health, sex and diet of the subject, the mode and time of administration, rate of excretion, drug combination, and severity of the particular condition. Subjects to be treated may include animals, or mammalian species such as humans.

The Hsp70 complexes of the present invention may be employed alone or in combination with each other and/or other suitable therapeutic agents, such as antiinflammatories, antiproliferatives, chemotherapeutic agents, and immunosuppressants.

The following examples are meant to be illustrative of an embodiment of the present invention and do not limit the scope of the invention in any way.

### Manufacture of Recombinant Hsp70

The following method was used to make recombinant Hsp70: Hsp70 fusion proteins were generated by PCR amplification of human Hsp70 DNA sequences using primers corresponding to the published sequence and including restriction endonuclease sites to enable directed cloning into a prokaryotic expression vector, ProExHta™ (Life Technologies, Inc.). NF-kB p50 sequences were generated in the same way, using primers corresponding to the published p50 sequence and cloned upstream of (5' to) Hsp70 sequences in the same expression vector. The cloning vector included a 6x His tag for use in purification of expressed protein over a metal column. DNA was transformed into a bacterial host and protein expression was induced using IPTG. Soluble, expressed fusion protein was purified using conventional affinity purification techniques and subsequently used for the following experiments.

### Example 1

### Hsp70-mediated Transport is Cell-Type Specific

To investigate the intracellular localization of exogenously added Hsp70, we fluorescently labeled the full-length Hsp70 protein and tested its ability to become internalized by various cell types. Cells were treated with a final concentration of 10 µg/ml Hsp70-FITC (or BSA-FITC as a control) for 1 hour, then washed and fixed prior to confocal laser scanning microscopy. The bulk of the intracellular Hsp70-FITC localized uniformly to the nucleus and cytoplasm in murine pre-B 70Z/3 cells, whereas import of BSA-FITC was negligible (Figure 1A and 1B). We also observed significant uptake of Hsp70-FITC by human peripheral blood monocytes, but not T cells (Figures 1C-1F). Interestingly, while the staining pattern in peripheral blood monocytes was often uniform throughout the cytoplasm and nucleus, we noted that it was sometimes characterized by punctuate staining, implying vesicular localization within the cytoplasm. Applicants found that the specific staining pattern of the PBLs varied by donor, suggesting that the state of cell activation may play a role in uptake efficiency or intracellular localization of exogenous Hsp70. Consistent with this hypothesis, we found that while resting peripheral blood B cells were resistant to uptake, they could be induced to transport the protein after 48 hours of activation *in vitro* with anti-CD40 plus anti-Ig antibodies. This method of B cell activation is known to result in the expression of various differentiation and proliferation associated genes. In contrast, activation of peripheral blood T cells by anti-CD3 and anti-CD28 antibodies did not affect Hsp70 transport. No intracellular uptake was observed by Jurkat T cell line or HeLa fibroblast cell line, but we did observe efficient uptake by two mature B cell lines, RAJI and BJAB. In addition, we saw limited cytoplasmic uptake by two monocytic cell lines, THP-1 and U937, but only after extended (6 to 24 hours) incubation with high concentrations (100 ug/ml) of Hsp70-FITC. The B cell- and monocyte-specific Hsp70 transport activity we describe is consistent with reports which propose a role for Hsp70 heat shock family members in antigen presentation (Manara, et al., (1993) Blood 82:2865-2871; Vanbuskirk, et al., (1989) J. Exp. Med. 170:1799-1809) as these cells are generally considered to function as antigen presenting cells. The cell type specificity and inducibility of cellular Hsp70 uptake may reflect differential expression of a required surface or nuclear receptor for the Hsp70 protein. Studies are now in progress to investigate the surface proteins which may be involved in the binding and internalization of extracellular Hsp70.

### Example 2

### Kinetics of Hsp70 Cellular Uptake

We next investigated the time-course and dose effect of Hsp70 uptake by 70Z/3 cells. 5x10⁵ cells were incubated at 37°C with 1 µg/ml Hsp70-FITC for increasing periods of time from 10 minutes to 48 hours. After unincorporated Hsp70-FITC was washed away with PBS, intracellular and cell-associated fluorescence was quantified by fluorimeter analysis. From these studies we determined that the internalization process appears to be slow, since maximal internalization is not achieved until between 6-8 hours (Figure 2A), after which time the rate of uptake appears to drop slightly and remain constant for up to 2 days of incubation.

For the dose range study we chose a one-hour incubation time and treated cells with varying amounts of Hsp70-FITC. Our analysis showed that the intensity of intracellular fluorescence increased with increasing concentrations of extracellular Hsp70, up to 100 µg/ml (1.4 µM), and was detectable even when cells were treated with levels as low as 0.1 µg/ml (1.4 nM, Figure 2B). We determined that treatment of cells with an extracellular concentration of 1 µM Hsp70-FITC resulted after one hour of incubation in an intracellular concentration of 700 nM, assuming a volume of 1 pl/cell. This uptake efficiency is comparable to that reported for other peptide sequences (Phelan, et al., (1998) Nature Biotechnology 16:440-443). The uptake of Hsp70-FITC was not saturable in the concentration range we used (35 nM-1 µM), indicative of a high capacity receptor-mediated uptake mechanism. In addition, internalization could not be blocked by preincubation with a 10-fold excess of unlabeled Hsp70.

### Example 3

### Mechanism of Hsp70-Mediated Intracellular Transport

To investigate the mechanism of transport, we examined the import of Hsp70 under various conditions. Although full-length Hsp70 protein was transported by PBLs in the presence of 0.05% sodium azide, this transport activity was completely eradicated when cells were incubated at 4°C (Figure 3A-3D), suggesting that there is an energy-dependent component to the Hsp70-mediated transport. These data coupled with co-internalization studies using transferrin, which revealed that accumulation of transferrin and Hsp70 occurred in separate intracellular compartments in both 70Z/3 cells (Figure 3E) and in human PBLs, suggested that this mechanism does not involve classical endocytosis.

### Example 4

### An Exogenous NF-κB-C terminal Hsp70 Fusion Protein Can Be Directed to the Nucleus

Two different fusion proteins composed of either a 244 (SEQ ID NO:2) or a 92 (SEQ ID NO:3) amino acid Hsp70 C-terminal polypeptide fused to the p50 subunit of the transcription factor NF-κB were generated to examine the ability of the Hsp70 peptide sequence to direct other protein substrates into the cell. The 244 amino acid polypeptide has the following sequence:

PLSLGLETAG GVMTALIKRN STIPTKQTQI FTTYSDNQPG VLIQVYEGER
AMTKDNNLLG RFELSGIPPA PRGVPQIEVT FDIDANGILN VTATDKSTGK
ANKITITNDK GRLSKEEIER MVQEAEKYKA EDEVQRERVS AKNALESYAF
NMKSAVEDEG LKGKISEADK KKVLDKCQEV ISWLDANTLA EKDEFEHKRK
ELEQVCNPII SGLYQGAGGP GPGGFGAQGP KGGSGSGPTI EEVD

The 92 amino acid polypeptide has the following sequence:

KSAVEDEGLK GKISEADKKK VLDKCQEVIS WLDANTLAEK DEFEHKRKEL
EQVCNPIISG LYQGAGGPGP GGFGAQGPKG GSGSGPTIEE VD

When added exogenously to cells, both FITC-conjugated fusion proteins entered the cytoplasm and nuclei of 70Z/3 cells (Figure 4) and PBLs (data not shown) with kinetics and specificity similar to the Hsp70 peptide alone. This transport was stable, nonsaturable and occurred without significant protein degradation. Maximal uptake of FITC-Hsp70 remained stable after a 30 minute pulse for 24 hours after washout of unincorporated protein and incubation at 37°C, as determined by confocal microscopy and SDS-PAGE analysis. To assess protein stability, cells were pulsed with either 10 µg/ml FITC-conjugated full-length Hsp70 or Hsp70/28-p50 for 1 hour prior to washing and a chase at 37°C for up to 96 hours. Whole cell extracts were generated by lysis in Laemmli sample buffer, and proteins were separated by SDS-PAGE. Gels were subjected to analysis by fluorimager, and results showed the presence of both the ~75 kD fusion protein as well as full-length Hsp70 itself (distinguishable from endogenous Hsp70 by its fluorescent tag) in whole cell extracts after up to 24 hours of incubation without measurable change in size or appearance of smaller molecular weight degradation products (Figure 5). These data suggest that intracellular targeted Hsp70 was retained by the cell without significant degradation with a half-life of greater than 48 hours.

### Example 5

### Transported NF-κB p50 Exhibits DNA-Binding Activity

To address whether the internalized fusion proteins retained functional activity, we tested nuclear extracts of Hsp70-p50 fusion protein-treated cells for their ability to bind a specific kappa DNA sequence. We could show that purified fusion proteins were able to bind kappa DNA (data not shown), suggesting that the p50 subunits were not conformationally impaired by the presence of the Hsp70 sequences. After 70Z/3 cells were treated with 100 ng/ml LPS, 10 µg/ml Hsp70/10-p50 or Hsp70/28-p50 for 1 hour, nuclear extracts were prepared and gel shift assays were performed. We found that DNA binding activity was retained by the fusion protein after nuclear uptake by cells, indicating that the import process did not result in significant degradation or loss of activity (Figure 6A). This DNA binding activity was specific, as the complex was competed with an excess of unlabeled NF-κB sequence but not with octamer sequence (Figure 6A). We saw distinct complexes formed by nuclear extracts from cells treated with different fusion proteins; furthermore, they differed from the complex formed by LPS-induced endogenous NF-κB. In supershift experiments we observed that anti-p50 antibodies were able to shift nearly the entire DNA-binding complex from fusion protein-treated cells, as expected, confirming that the fusion protein was likely binding to DNA mainly as a homodimer (Figure 6B). We also observed a detectable decrease in the specific complex upon incubation with both anti-p65 and anti-Hsp70 (directed against the carboxy-terminal four amino acids EEVD) antibodies, indicating the presence of endogenous p65 subunits in addition to recombinant Hsp70-p50 subunits. In contrast, nuclear extracts from control LPS-treated cells formed a complex containing both p50 and p65 subunits, and the supershifted patterns differed from the cells treated with the fusion protein. These data indicate that distinct protein/DNA complexes were formed and suggest that the fusion proteins were binding DNA directly and not simply activating endogenous NF-κB.

### Example 6

### Hsp70-p50 Fusion Protein Activates Surface kappa Ig Expression and TNFα Production

Since treatment of cells with Hsp70-p50 fusion proteins could potentially result in the formation of complexes which interact with NF-κB DNA binding sites in cells, we decided to evaluate downstream biological events in the NF-κB pathway. We observed that treatment of various cells with the Hsp70-p50 fusion proteins resulted in activation of several inflammatory and immunological responses normally regulated by NF-κB. Treatment of mouse 70Z/3 pre-B cells with Hsp70/10-p50 fusion protein was shown to be as effective as LPS in inducing high levels of kappa Ig light chain on the surface (Figure 7A). In addition, in contrast to LPS-induced activation, the fusion protein-induced surface kappa expression was abolished by 30 minutes of 65°C heat denaturation of the protein prior to treatment of cells, confirming that intact protein was required for the activation. Similar results were obtained with Hsp70/28-p50 (data not shown). TNFα production is another example of an inflammatory response also largely regulated by NF-κB. We observed that the internalized fusion protein was also able to induce TNFα production by human peripheral blood lymphocytes (Figure 7B). Freshly isolated PBLs were incubated with LPS or Hsp70-p50 for 6 hours, after which time supernatants were collected and tested for cytokine levels by ELISA. Again, we found the fusion proteins to be as effective as LPS in inducing TNFα production, and established that intact protein was responsible for activation by showing that heat denaturation of the fusion protein abolished the effect.

The following experimental procedures were used in the above examples:

**Expression and purification of the Hsp70-p50 fusion proteins.** Two p50 fusion proteins were constructed using the nucleotide sequence corresponding to amino acids 1-406 of the NF-κB p105 subunit protein. This sequence includes the DNA binding domain as well as the rel homology domain. The two fusion proteins varied in the length of Hsp70 fragment used. The two Hsp70 sequences were both derived from the C-terminus, including either the terminal 276 or 735 nucleotides, which correspond to a 10 kD (the 92 amino acid polypeptide discussed above) and a 28 kD (the 244 amino acid polypeptide discussed above) protein fragment. Either the 10 kD or the 28 kD Hsp70 protein was fused C-terminal to the p50 protein, and the resulting fusion proteins were denoted Hsp70/10-p50 or Hsp70/28-p50; respectively. The prokaryotic expression vector ProEX HT™ (Life Technologies, Gaithersburg, MD) was used for cloning, expression and purification, as per the manufacturer's recommendations.

**Confocal laser scanning microscopy**. Cells were typically treated with 10 µg/ml FITC-conjugated proteins or as indicated in the text for one hour at 37°C followed by a wash in PBS, fixation in 2% paraformaldehyde, an additional wash in PBS and subsequent visual analysis by confocal microscopy (Bio-Rad, Hercules, CA) using Molecular Dynamics LaserSharp software and Adobe® Photoshop®.

**Western blot analysis of imported Hsp70-p50** fusion proteins. Cells were treated with FITC-conjugated proteins for one hour at 37°C, washed in PBS and used for preparation of nuclear extracts. Equal protein amounts were separated by SDS-PAGE. The gel was fixed in acetic acid and subject to fluorescence analysis by fluorimager and ImageQuant software.

**Electrophoretic mobility shift assay.** Nuclear extracts from 70Z/3 cells were prepared using a modification of established protocols (Tepper, et al., (1995) J. Immunol. 155:2427-2436). Protein concentrations were determined using the Bradford assay, and NF-κB (5'GATCCGAGGGGACTTTCCGCTGGGGACTTTCCAGG3' (SEQ ID NO:4)) or octamer (5'TGTCGAATGCAAATCACTAGAA3' (SEQ ID NO:5)) oligonucleotides (Promega, Madison, WI) were end labeled with [γ-³²P)ATP and T4 kinase. The conditions for binding reactions with oligonucleotide probes were as previously described. Supershift assays were performed with NF-κB p50, p65 and c-Rel polyclonal antibodies (Santa Cruz Biotechnology, Santa Cruz, CA) and Hsp70 antibody by preincubating the nuclear extracts with 3 ul of the antibody in the reaction buffer for 30 minutes and continuing with the gel retardation assay according to standard procedures. Competition experiments were performed using unlabeled NF-κB and octamer oligonucleotides. Samples were analyzed on native 6% polyacrylamide gels and autoradiographed.

**Immunofluorescence assay (FACS).** 70Z/3 cells were treated with either 30 µg/ml Hsp70/10-p50 fusion protein or 100 ng/ml LPS and incubated overnight at 37°C. Cells were then washed in PBS and fixed in 2% paraformaldehyde prior to staining with FITC-conjugated anti-kappa antibody. After an additional PBS wash, cells were subjected to imaging and analysis on the FACSTAR™.

**TNFα assay.** Human peripheral blood lymphocytes were isolated as previously described and treated with 40 µg/ml Hsp70 fusion protein or LPS for 6 hours. Supernatants were collected and analyzed for TNFα by ELISA (Genzyme, Cambridge, MA).

## Claims

1. An in vitro method for delivering a compound selected from the group consisting of a protein, a peptide, a polynucleotide or a therapeutic agent into the nucleus of a cell or cells, said method comprising joining said compound with Hsp70, or a fragment of Hsp70 comprising an amino acid sequence as shown in SEQ ID NO: 2 or SEQ ID NO: 3, to form an Hsp70 complex, and exogenously providing said Hsp70 complex to said cell or to the environment surrounding said cell so that Hsp70 complex is imported into the nucleus of said cell or cells.

2. Use of a Hsp70 complex for the preparation of a pharmaceutical composition for treating transplant rejection, an autoimmune disease, an inflammatory disease, cancer, or a vascular disease in a patient, wherein a compound is covalently joined with Hsp70, or a fragment of Hsp70 comprising an amino acid sequence as shown in SEQ ID NO: 2 or SEQ ID NO: 3, to form the Hsp70 complex and wherein said complex is to be imported into the nucleus of a cell or cells.

3. The use of claim 2 wherein said Hsp70 complex comprises a compound selected from the group consisting of a protein, a peptide, a polynucleotide, and a therapeutic agent.

4. The in vitro method of claim 1 for delivering a protein or a peptide to the nuclear compartment of a cell or cells, said method comprising joining said protein or peptide with Hsp70 or a fragment of Hsp70 comprising an amino acid sequence as shown in SEQ ID NO: 2 or SEQ ID NO: 3 to form an Hsp70 complex, and exogenously providing said complex to said cell or cells so that said complex is transported into the nuclear compartment of said cell or cells.

5. The use of claim 2, wherein a nuclear protein or nuclear peptide is joined with Hsp70, or a fragment of Hsp70 comprising an amino acid sequence as shown in SEQ ID NO: 2 or SEQ ID NO: 3 to form the Hsp70 complex.

6. The method of claim 4 or the use of claim 5, wherein said fragment of Hsp70 comprises an amino acid sequence as shown in SEQ ID NO:2 or SEQ ID NO:3.

7. The method of claims 4 or 6, or the use of claims 3 or 5 wherein said protein or peptide comprises the p50 subunit of the transcription factor NF-κB.

8. A pharmaceutical composition comprising an Hsp70 complex, said Hsp70 complex comprising a first portion and second portion, said first portion comprising Hsp70 protein, or a fragment of Hsp70 comprising an amino acid sequence as shown in SEQ ID NO: 2 or SEQ ID NO:3; and said second portion comprising a nuclear protein, nuclear peptide, or polynucleotide.

9. The pharmaceutical composition of claim 8, wherein the Hsp70 complex comprises Hsp70 protein of human origin.

10. The pharmaceutical composition of claim 8 or 9, wherein said protein or peptide comprises the p50 subunit of the transcription factor NF-κB.

11. The method or the use of claim 6, wherein said fragment of Hsp70 consists of the amino acid sequence shown as SEQ ID NO:2, or in SEQ ID NO:3.

12. The pharmaceutical composition of claim 8 or 9 further comprising at least one additional active compound selected from the group consisting of an immunosuppressant, an anti-cancer agent, an anti-viral agent, an anti-inflammatory agent, an anti-fungal agent, an antibiotic, and an anti-vascular hyperproliferative compound.

13. The pharmaceutical composition of claim 8 or 9 or the method of claim 4 comprising a nuclear protein or nuclear peptide covalently joined to said Hsp70 protein or a fragment of Hsp70 comprising an amino acid sequence as shown in SEQ ID NO: 2 or SEQ ID NO: 3.

14. The pharmaceutical composition of claim 8, 9 or 13, wherein said Hsp70 or fragment of Hsp70 comprises an amino acid sequence as shown in SEQ ID NO:2 or SEQ ID NO:3.

15. The pharmaceutical composition of claim 13 or 14, wherein said Hsp70 or a fragment of Hsp70 comprising an amino acid sequence as shown in SEQ ID NO: 2 or SEQ ID NO: 3 is C-terminal to said protein or peptide.

16. The pharmaceutical composition of claim 10, wherein said protein or peptide comprises residues 37-409 of the p50 subunit of the transcription factor NF-κB.

17. The pharmaceutical composition of any one of claims 8, 9, 12, 13 or 14 for treating transplant rejection, an autoimmune disease, or an inflammatory disease in a patient.

18. The method of claim 13, the use of claim 5, or the pharmaceutical composition of claims 8 or 13, wherein said nuclear protein or nuclear peptide is capable of modulating gene expression in said nuclear compartment.

19. The method, use, or pharmaceutical composition of claim 18, wherein said modulation of gene expression provides immunosuppressive or immunostimulatory control.

20. The method, use, or pharmaceutical composition of claim 18, wherein said nuclear protein or nuclear peptide is a transcription factor.

## Patentansprüche

1. In-vitro-Verfahren zum Zuführen einer Verbindung, ausgewählt aus der Gruppe bestehend aus einem Protein, einem Peptid, einem Polynukleotid oder einem therapeutischen Mittel, in den Kern von einer Zelle oder von Zellen, wobei das Verfahren umfaßt, die Verbindung mit Hsp70 oder einem Fragment von Hsp70, umfassend eine Aminosäuresequenz wie gezeigt in SEQ ID NO: 2 oder SEQ ID NO: 3, zu verknüpfen, um einen Hsp70-Komplex zu bilden, und den Hsp70-Komplex der Zelle oder dem Umfeld, umgebend die Zelle, exogen bereitzustellen so daß der Hsp70-Komplex in den Kern von der Zelle oder den Zellen eingeführt wird.

2. Verwendung eines Hsp70-Komplexes für die Herstellung einer pharmazeutischen Zusammensetzung zum Behandeln von Transplantatabstoßung, einer Autoimmunkrankheit, einer Entzündungskrankheit, Krebs oder einer Gefäßkrankheit bei einem Patienten, wobei eine Verbindung mit Hsp70 oder einem Fragment von Hsp70, umfassend eine Aminosäuresequenz wie gezeigt in SEQ ID NO: 2 oder SEQ ID NO: 3, kovalent verknüpft wird, um den Hsp70-Komplex zu bilden, und wobei der Komplex in den Kern von einer Zelle oder von Zellen eingeführt werden soll.

3. Verwendung nach Anspruch 2, wobei der Hsp70-Komplex eine Verbindung, ausgewählt aus der Gruppe bestehend aus einem Protein, einem Peptid, einem Polynukleotid und einem therapeutischen Mittel, umfaßt.

4. In-vitro-Verfahren nach Anspruch 1 zum Zuführen eines Proteins oder eines Peptids zu dem nukleären Kompartiment von einer Zelle oder von Zellen, wobei das Verfahren umfaßt, das Protein oder Peptid mit Hsp70 oder einem Fragment von Hsp70, umfassend eine Aminosäuresequenz wie gezeigt in SEQ ID NO: 2 oder SEQ ID NO: 3, zu verknüpfen, um einen Hsp70-Komplex zu bilden, und den Komplex der Zelle oder den Zellen exogen bereitzustellen, so daß der Komplex in das nukleäre Kompartiment von der Zelle oder den Zellen transportiert wird.

5. Verwendung nach Anspruch 2, wobei ein nukleäres Protein oder nukleäres Peptid mit Hsp70 oder einem Fragment von Hsp70, umfassend eine Aminosäuresequenz wie gezeigt in SEQ ID NO: 2 oder SEQ ID NO: 3, verknüpft wird, um den Hsp70-Komplex zu bilden.

6. Verfahren nach Anspruch 4 oder Verwendung nach Anspruch 5, wobei das Fragment von Hsp70 eine Aminosäuresequenz wie gezeigt in SEQ ID NO: 2 oder SEQ ID NO: 3 umfaßt.

7. Verfahren nach den Ansprüchen 4 oder 6 oder Verwendung nach den Ansprüchen 3 oder 5, wobei das Protein oder Peptid die p50-Untereinheit des Transkriptionsfaktors NF-κB umfaßt.

8. Pharmazeutische Zusammensetzung, umfassend einen Hsp70-Komplex, wobei der Hsp70-Komplex einen ersten Teil und einen zweiten Teil umfaßt, wobei der erste Teil Hsp70-Protein oder ein Fragment von Hsp70, umfassend eine Aminosäuresequenz wie gezeigt in SEQ ID NO: 2 oder SEQ ID NO: 3, umfaßt; und der zweite Teil ein nukleäres Protein, nukleäres Peptid oder Polynukleotid umfaßt.

9. Pharmazeutische Zusammensetzung nach Anspruch 8, wobei der Hsp70-Komplex Hsp70-Protein humanen Ursprungs umfaßt.

10. Pharmazeutische Zusammensetzung nach Anspruch 8 oder 9, wobei das Protein oder Peptid die p50-Untereinheit des Transkriptionsfaktors NF-κB umfaßt.

11. Verfahren oder Verwendung nach Anspruch 6, wobei das Fragment von Hsp70 aus der Aminosäuresequenz, gezeigt als SEQ ID NO: 2 oder in SEQ ID NO: 3, besteht.

12. Pharmazeutische Zusammensetzung nach Anspruch 8 oder 9, weiterhin umfassend mindestens eine zusätzliche aktive Verbindung, ausgewählt aus der Gruppe bestehend aus einem Immunsuppressivum, einem Antikrebsmittel, einem antiviralen Mittel, einem entzündungshemmenden Mittel, einem antifungalen Mittel, einem Antibiotikum und einer anti-vaskulär-hyperproliferativen Verbindung.

13. Pharmazeutische Zusammensetzung nach Anspruch 8 oder 9 oder Verfahren nach Anspruch 4, umfassend ein nukleäres Protein oder nukleäres Peptid, kovalent verknüpft mit dem Hsp70-Protein oder einem Fragment von Hsp70, umfassend eine Aminosäuresequenz wie gezeigt in SEQ ID NO: 2 oder SEQ ID NO: 3.

14. Pharmazeutische Zusammensetzung nach Anspruch 8, 9 oder 13, wobei das Hsp70 oder Fragment von Hsp70 eine Aminosäuresequenz wie gezeigt in SEQ ID NO: 2 oder SEQ ID NO: 3 umfaßt.

15. Pharmazeutische Zusammensetzung nach Anspruch 13 oder 14, wobei das Hsp70 oder ein Fragment von Hsp70, umfassend eine Aminosäuresequenz wie gezeigt in SEQ ID NO: 2 oder SEQ ID NO: 3, C-terminal zu dem Protein oder Peptid ist.

16. Pharmazeutische Zusammensetzung nach Anspruch 10, wobei das Protein oder Peptid die Reste 37-409 der p50-Untereinheit des Transkriptionsfaktors NF-κB umfaßt.

17. Pharmazeutische Zusammensetzung nach einem der Ansprüche 8, 9, 12, 13 oder 14 zum Behandeln von Transplantatabstoßung, einer Autoimmunkrankheit oder einer Entzündungskrankheit bei einem Patienten.

18. Verfahren nach Anspruch 13, Verwendung nach Anspruch 5 oder pharmazeutische Zusammensetzung nach Anspruch 8 oder 13, wobei das nukleäre Protein oder nukleäre Peptid imstande ist, die Genexpression in dem nukleären Kompartiment zu modulieren.

19. Verfahren, Verwendung oder pharmazeutische Zusammensetzung nach Anspruch 18, wobei die Modulation der Genexpression immunsuppressive oder immunstimulierende Kontrolle bereitstellt.

20. Verfahren, Verwendung oder pharmazeutische Zusammensetzung nach Anspruch 18, wobei das nukleäre Protein oder nukleäre Peptid ein Transkriptionsfaktor ist.

## Revendications

1. Procédé *in vitro* pour délivrer un composé sélectionné à partir du groupe consistant en une protéine, un peptide, un polynucléotide ou un agent thérapeutique, dans le noyau d'une cellule ou de cellules, ledit procédé comprenant le fait de joindre ledit composé à une Hsp70 ou à un fragment de Hsp70 comprenant une séquence d'acides aminés telle que présentée dans la SEQ ID NO:2 ou dans la SEQ ID NO:3, pour former un complexe Hsp70 et fournir de manière exogène ledit complexe Hsp70 à ladite cellule ou au milieu entourant ladite cellule de telle sorte que le complexe Hsp70 est importé dans le noyau de ladite cellule ou desdites cellules.

2. Utilisation d'un complexe Hsp70 dans la préparation d'une composition pharmaceutique pour traiter un rejet de greffe, une maladie auto-immune, une maladie inflammatoire, un cancer ou une maladie vasculaire chez un patient, où un composé est joint de manière covalente à une Hsp70 ou à un fragment de Hsp70 comprenant une séquence d'acides aminés telle que présentée dans la SEQ ID NO:2 ou dans la SEQ ID NO:3, pour former un complexe Hsp70 et où ledit complexe doit être importé dans le noyau d'une cellule ou de cellules.

3. L'utilisation de la revendication 2, où ledit complexe Hsp70 comprend un composé sélectionné à partir du groupe consistant en une protéine, un peptide, un polynucléotide et un agent thérapeutique.

4. Le procédé *in vitro* de la revendication 1 pour délivrer une protéine ou un peptide au compartiment nucléaire d'une cellule ou de cellules, ledit procédé comprenant le fait de joindre ladite protéine ou ledit peptide à une Hsp70 ou à un fragment de Hsp70 comprenant une séquence d'acides aminés telle que présentée dans la SEQ ID NO:2 ou dans la SEQ ID NO:3, pour former un complexe Hsp70 et fournir de manière exogène ledit complexe à ladite cellule ou auxdites cellules de telle sorte que ledit complexe est transporté dans le compartiment nucléaire de ladite cellule ou desdites cellules.

5. L'utilisation de la revendication 2, où une protéine nucléaire ou un peptide nucléaire est joint(e) à une Hsp70 ou à un fragment de Hsp70 comprenant une séquence d'acides aminés telle que présentée dans la SEQ ID NO:2 ou dans la SEQ ID NO:3, pour former le complexe Hsp70.

6. Le procédé de la revendication 4 ou l'utilisation de la revendication 5, où ledit fragment de Hsp70 comprend une séquence d'acides aminés telle que présentée dans la SEQ ID NO:2 ou dans la SEQ ID NO:3.

7. Le procédé des revendications 4 ou 6, ou l'utilisation des revendications 3 ou 5, où ladite protéine ou ledit peptide comprend la sous-unité p50 du facteur de transcription NF-κB.

8. Composition pharmaceutique comprenant un complexe Hsp70, ledit complexe Hsp70 comprenant une première portion et une deuxième portion, ladite première portion comprenant une protéine Hsp70 ou un fragment de Hsp70 comprenant une séquence d'acides aminés telle que présentée dans la SEQ ID NO:2 ou dans la SEQ ID NO:3 et ladite deuxième portion comprenant une protéine nucléaire, un peptide nucléaire ou un polynucléotide.

9. La composition pharmaceutique de la revendication 8, dans laquelle le complexe Hsp70 comprend une protéine Hsp70 d'origine humaine.

10. La composition pharmaceutique de la revendication 8 ou 9, dans laquelle ladite protéine ou ledit peptide comprend la sous-unité p50 du facteur de transcription NF-κB.

11. Le procédé ou l'utilisation de la revendication 6, où ledit fragment de Hsp70 consiste en la séquence d'acides aminés présentée dans la SEQ ID NO:2 ou dans la SEQ ID NO:3.

12. La composition pharmaceutique de la revendication 8 ou 9, comprenant en outre au moins un composé actif supplémentaire sélectionné à partir du groupe consistant en un immunosuppresseur, un agent anticancéreux, un agent antiviral, un agent anti-inflammatoire, un agent antifongique, un antibiotique et un composé d'anti-hyperprolifération vasculaire.

13. La composition pharmaceutique de la revendication 8 ou 9 ou le procédé de la revendication 4, comprenant une protéine nucléaire ou un peptide nucléaire joint(e) de manière covalente à ladite protéine Hsp70 ou à un fragment de Hsp70 comprenant une séquence d'acides aminés telle que présentée dans la SEQ ID NO:2 ou dans la SEQ ID NO:3.

14. La composition pharmaceutique de la revendication 8, 9 ou 13, dans laquelle ladite Hsp70 ou fragment de Hsp70 comprend une séquence d'acides aminés telle que présentée dans la SEQ ID NO:2 ou dans la SEQ ID NO:3.

15. La composition pharmaceutique de la revendication 13 ou 14, dans laquelle ladite Hsp70 ou un fragment de Hsp70 comprenant une séquence d'acides aminés telle que présentée dans la SEQ ID NO:2 ou dans la SEQ ID NO:3 est C-terminale à ladite protéine ou audit peptide.

16. La composition pharmaceutique de la revendication 10, dans laquelle ladite protéine ou ledit peptide comprend les résidus 37-409 de la sous-unité p50 du facteur de transcription NF-κB.

17. La composition pharmaceutique de l'une quelconque des revendications 8, 9, 12, 13 ou 14, pour traiter un rejet de greffe, une maladie auto-immune ou une maladie inflammatoire chez un patient.

18. Le procédé de la revendication 13, l'utilisation de la revendication 5 ou la composition pharmaceutique des revendications 8 ou 13, où ladite protéine nucléaire ou ledit peptide nucléaire est capable de moduler l'expression génique dans ledit compartiment nucléaire.

19. Le procédé, l'utilisation ou la composition pharmaceutique de la revendication 18, où ladite modulation de l'expression génique fournit un contrôle immunosuppresseur ou immunostimulateur.

20. Le procédé, l'utilisation ou la composition pharmaceutique de la revendication 18, où ladite protéine nucléaire ou ledit peptide nucléaire est un facteur de transcription.
